# NEUE EUROPÄISCHE PATENTSCHRIFT

(11) **EP 0 764 423 B2**
(45) Veröffentlichungstag und Bekanntmachung des Hinweises auf die Entscheidung über den Einspruch: **03.03.2010**
(45) Hinweis auf die Patenterteilung: 29.09.2004
(21) Anmeldenummer: 96111708.2
(22) Anmeldetag: 19.07.1996
(51) Int. Cl.: A61B 1/00

(54) **Abwinkelbares Rohr**
Bendable pipe
Tube pliable

(30) Priorität: 22.09.1995 DE 19535179
(43) Veröffentlichungstag der Anmeldung: 26.03.1997
(73) Patentinhaber: Richard Wolf GmbH, 75438 Knittlingen (DE)
(72) Erfinder: Heimberger, Rudolf, 75038 Oberderdingen (DE)
(74) Vertreter: Vollmann, Heiko

(56) Entgegenhaltungen:
- EP-A- 0 612 496
- EP-A- 0 626 604
- DE- - 1 816 973
- DE-A- 2 618 732
- DE-C- 4 222 121
- US- - 2 515 366

## Beschreibung

Die Erfindung betrifft ein abwinkelbares Rohr, insbesondere als Schaft für ein flexibles Endoskop.

Fiberskope nach dem Stand der Technik weisen am distalen Ende einen hochflexiblen, beliebig ablenkbaren Abschnitt auf, um in einer Körperhöhle oder dergleichen beispielsweise ausgewählte Gewebebereiche gezielt visuell kontrollieren und/oder mittels zusätzlicher Hilfsinstrumente therapieren zu können.

Weiterhin sind Schäfte mit einem biegsamen Abschnitt bei im Körperinneren wirksamen Schneid- oder Fräsinstrumenten in der minimal invasiven Chirurgie bekannt. Bei letzteren soll trotz seitlich abgewinkeltem Schneidkopf ein hohe Kraftübertragung möglich sein, da das zu bearbeitende Material, z.B. Knochensubstanz unter Umständen hart und zäh sein kann.

Bei dem aus DE-GM 69 38 905 bekannten Endoskopschaft besteht ein flexibler Abschnitt eines aus einer Vielzahl axial aneinandergereihter, miteinander gelenkig verbundener Ringglieder. Bei der Herstellung müssen die Gelenkverbindungen zwischen den einzelnen Ringabschnitten separat angebracht werden. Ein weiterer, aus der DE-PS 17 66 209 bekannter flexibler Endoskopschaft weist lose auf vier Steuerdrähten aneinandergereihte d.h. nicht formschlüssig miteinander in Eingriff stehende Zylinderabschnitte auf.

Eine weitere aus der DE-PS 18 16 973 bekannte biegsame Endoskoprohranordnung besteht aus hintereinander angeordneten Rohrabschnitten, die mittels Vorsprüngen und Aussparungen ohne Formschluß ineinandergreifen, jedoch voneinander lösbar sind. Aus EP 0 612 496 A1 ist ein ähnlicher Schaft für medizinische Instrumente bekannt, der aus hintereinander angeordneten Rohrabschnitten besteht, die teilweise durch Achsen und teilweise durch Vorsprünge und Ausnehmungen miteinander formschlüssig verbunden sind. Allerdings bedingt dort der nur Teilformschluss, dass zusätzlich ein Kraftschluss über in den Rohrabschnitten geführten Spanndrähten erforderlich ist, um die Rohrabschnitte aneinander festzuhalten. Ein aus EP 0 626 604 A2 bekanntes flexibles Endoskoprohr ist aus Rohrabschnitten aufgebaut, die abwechselnd um 90° versetzt zueinander Nasen- und Ausnehmungspaare aufweisen, welche die Rohrabschnitte formschlüssig miteinander verbinden. Auch dort sind zum Erhalt des Verbundes Spanndrähte vorgesehen, über die zugleich auch die Steuerung der Ablenkgröße und Richtung des Endoskoprohrs eingestellt wird.

Schließlich ist aus der DE-OS 24 47 510 ein flexibler Endoskopschaft bekannt, der ein zylindrisches Geflecht aufweist, auf dessen Außenseite eine Vielzahl von miteinander in Eingriff stehenden Ablenkelementen angeordnet sind. Jedes Ablenkelement weist an einem Ende einen Vorsprung und am anderen Ende eine Ausnehmung auf.

Bei allen oben angeführten, bekannten, flexiblen Schäften besteht der Nachteil, daß ihr Aufbau zum einen relativ aufwendig und zum anderen nur in Kombination mit weiteren Teilen für den bestimmungsgemäßen Gebrauch gefertigt werden kann. Diese bekannten, flexiblen Endoskopschäfte haben somit auch den wesentlichen Nachteil, daß ihre Fertigung aufwendig und wegen ihres konstruktiven Aufbaus kostenintensiv ist. Bei der Fertigung dieser bekannten, flexiblen Endoskopschäfte besteht außerdem der Nachteil, daß eine Vielzahl von Einzelbauteilen gefertigt, montiert und gelagert werden muß.

Bei einem aus der US-PS 51 52 744 bekannten, einen flexiblen Abschnitt aufweisenden Endoskopschaft wird die elastische Verformbarkeit des an sich starren Schaftes durch wechselweise in der Wandung angeordnete Ausnehmungen erreicht, die so geartet sind, daß die einzelnen Abschnitte über Stege einer bestimmten Breite materiell miteinander in Verbindung stehen. Dabei besteht jedoch die Gefahr, daß eine zu starke Verbiegung des flexiblen Abschnitts einen Bruch eines oder mehrerer Verbindungsstege hervorruft.

Aufgabe der vorliegenden Erfindung ist es, ein abwinkelbares Rohr, insbesondere als Schaft für ein flexibles Endoskop zu schaffen, das einerseits die eingangs erörterten Nachteile des Standes der Technik vermeidet und andererseits eine einfache und somit wirtschaftliche Fertigung gestattet. Weiterhin sollen zusätzliche Mittel zum Festlegen einzelner Rohrabschnitte entfallen, um den verbleibenden Freiraum innerhalb des Rohres möglichst vollständig für andere Kanäle, optische Einrichtungen, mechanische Einrichtungen und dergleichen nutzen zu können.

Der vorrichtungsmäßige Teil der obigen Aufgabe wird durch ein abwinkelbares Rohr gemäß den im Anspruch 1 aufgeführten Merkmalen gelöst. Die Erfindung sieht demgemäß ein abwinkelbares Rohr, insbesondere als Schaft für ein flexibles Endoskop vor, das aus Rohrabschnitten aufgebaut ist, wobei benachbarte Rohrabschnitte durch eine umlaufende Trennfuge materiell voneinander getrennt, jedoch formschlüssig mindestens in Richtung der Längsachse des Rohres miteinander verbunden sind, und ein Rohrabschnitt mindestens vier oder mehr zum benachbarten Rohrabschnitt weisende Nasen aufweist, wobei mindestens eine Nase in die entsprechende Ausnehmung des benachbarten Rohrabschnitts formschlüssig eingreift.

Dabei ist mindestens einer, vorzugsweise jedoch jeder Nase eine Ausnehmung im benachbarten Rohrabschnitt zugeordnet, wodurch einerseits eine gute Beweglichkeit in allen Richtungen trotz formschlüssiger Verbindung gewährleistet wird und andererseits die Stabilität des so gebildeten Rohres im Vergleich zum Stand der Technik erheblich verbessert wird. Vorteilhaft ist jede Ausnehmung durch den zwischen zwei benachbarten Nasen desselben Rohrabschnitts gebildeten Zwischenraum gebildet, so dass sich im Verbund zwischen benachbarten Rohrabschnitten Nasen und Ausnehmungen aneinander anschließen und somit einen Formschluss in sämtliche Richtungen bilden.

Das erfindungsgemäße abwinkelbare Rohr kann je nach Anordnung und Ausbildung der Trennfuge praktisch beliebig abwinkelbar ausgebildet sein. Der Formschluß zwischen benachbarten Rohrabschnitten sorgt dafür, daß Torsions-, Zug- und Biegekräfte zuverlässig aufgenommen werden können, es ergeben sich also bei entsprechenden Auslegung der Trennfuge, wie sie beispielhaft in den Unteransprüchen beansprucht und in der nachfolgenden Beschreibung beschrieben ist, abwinkelbare Rohre für nahezu jeden beliebigen Einsatzzweck. So ist es bei entsprechender Auslegung der Formschlußelemente möglich, die Abwinkelung nur in einer oder auch in mehreren Ebenen zu ermöglichen. So kann die Abwinkelung auch als Drehung des Rohres in sich ermöglicht werden.

Ein nach dem erfindungsgemäßen Prinzip aufgebautes abwinkelbares Rohr kann jedoch nicht nur als Schaft für ein flexibles Endoskop, sondern auch für zahlreiche andere Anwendungen eingesetzt werden. Im Bereich der Endoskoptechnik kann ein solches Rohr beispielsweise als Trokarhülse eingesetzt werden, die beim eigentlichen Einstechvorgang durch den darin befindlichen Trokar starr ist und erst nach Entfernen des Trokars flexibel wird. Weiterhin kann ein solches Rohr als Armierung für einen flexiblen Schlauch außen oder innen eingesetzt werden, es kann als Kardanersatz dienen oder auch als Hochfrequenzelektrode ausgebildet sein. Ein solches Rohr kann auch als Kette, nämlich als Hohlkette Verwendung finden. Auch ist ein Einsatz als Stent, z. B. als Gefäßstütze, als Speiseröhren- oder Luftröhrenstütze möglich. Der durch zueinander abwinkelbare Rohrabschnitte gebildete flexible Rohrteil kann durch eine Vielzahl miteinander im Eingriff stehender identischer Rohrabschnitte gebildet sein, was hinsichtlich Fertigungstechnik und Lagerhaltung besonders günstig sein wird. Es können aber auch unterschiedliche Rohrabschnitte miteinander kombiniert werden, und zwar nicht nur hinsichtlich der axialen Länge, sondern auch hinsichtlich der Ausbildung der Formschlußelemente.

Gemäß der Erfindung weist ein abwinkelbarer Rohrabschnitt mindestens vier zum benachbarten Rohrabschnitt weisende Nasen auf, die in entsprechende Ausnehmungen des benachbarten Rohrabschnitts formschlüssig eingreifen. Zur Bildung eines flexiblen Rohres sind mehrere solcher zueinander abwinkelbarer Rohrabschnitte erforderlich. Diese von zwei Rohrabschnitten benachbarten Rohrabschnitte weisen in Achsrichtung des Rohrs gesehen zu einer Seite mindestens vier Nasen und zur anderen Seite vier entsprechende Ausnehmungen auf.

Es können auch mehr als vier Nasen für jeden Rohrabschnitt vorgesehen sein, die in einer entsprechenden Anzahl von Ausnehmungen des benachbarten Rohrabschnitts eingreifen. Bevorzugt sind auch hierbei die Nasen so angeordnet, dass zwischen benachbarten Nasen wiederum eine Ausnehmung gebildet wird, in die eine Nase des benachbarten Rohrabschnitts eingreifen kann. Auf diese Weise wird eine innige Verzahnung benachbarter Rohrabschnitte in konstruktiv einfacher Weise erreicht. Die Form der einzelnen Nasen und korrespondierenden Ausnehmungen hängt von ihrer Funktion ab. Es werden mindestens vier pilzförmige Nasen vorzusehen sein, um zuverlässig einen Formschluss zu dem benachbarten Rohrabschnitt mit entsprechenden Ausnehmungen, in denen diese Nasen liegen, sicherzustellen. Die Flexibilität des Rohres kann einerseits durch die Form der Nase und andererseits durch die Breite der Trennfuge bestimmt werden. Um eine gezielte Abwinkelung zu ermöglichen, ist es zweckmäßig, die pilzförmige Nase mit einer zumindest abschnittsweise teilkreisförmigen Außenkontur zu versehen.

Um die Torsionsfestigkeit eines solchen Rohres zu erhöhen und die Abwinkelung beispielsweise nur in einer Ebene zuzulassen, ist es zweckmäßig, neben den vorerwähnten pilzförmigen Nasen eine oder mehrere rechteckige Nasen vorzusehen, die mit rechteckigen Ausnehmungen korrespondieren und in diese eingreifen. Durch solche Führungsnasen muß jedoch die Abwinkelbarkeit nicht zwingend richtungsbeschränkt werden, wenn diese in Längsachse des Rohres gesehen versetzt zueinander angeordnet werden. Der Versatz kann in einfacher Weise dadurch erreicht werden, daß an jedem Rohrabschnitt die Ausnehmungen zu den korrespondierenden Nasen um einen gewissen Winkel versetzt angeordnet sind. Es können jedoch auch Gruppen unterschiedlicher Rohrabschnitte vorgesehen sein, die alternierend miteinander im Eingriff stehen.

Gemäß einer Weiterbildung der Erfindung kann durch entsprechende Formgebung der Nasen und Ausnehmungen nicht nur ein abwinkelbares bzw. flexibles Rohr geschaffen werden, sondern darüberhinaus auch in seiner Länge veränderbares Rohr, wenn nämlich zwischen miteinander in Eingriff stehenden Nasen und Ausnehmungen im Hinterschneidungsbereich ein definiertes Spiel in axialer Richtung vorgesehen ist. Ein solcher axialer Längenausgleich kann beispielsweise dann zweckmäßig sein, wenn das Rohr als flexible Welle eingesetzt wird und ein Axialausgleich ähnlich einer Kerbverzahnung oder dergleichen erforderlich ist.

In einer Weiterbildung der Erfindung können die pilzförmigen Nasen und entsprechend konturierten Ausnehmungen schräg zur Längsachse des Rohres ausgerichtet sein, derart, daß sich eine gedachte axiale Verlängerung von Nasen bzw. Ausnehmungen schraubenlinienförmig um das Rohr erstreckt. Wenn bei solch einer Ausführung wiederum ein definiertes Spiel zwischen Nasen und Ausnehmungen im Bereich der Hinterschneidungen vorgesehen wird, so kann durch Anordnung von Zugmitteln und/oder Druckmitteln innerhalb oder außerhalb des Rohres eine Drehbewegung durch Erzeugung einer Axialbewegung erreicht werden. Es kann also durch Steuerung der axialen Rohrlänge der Verdrehwinkel zwischen Anfang und Ende des Rohres gezielt gesteuert werden.

Die vorbeschriebenen Rohrabschnitte zur Bildung des erfindungsgemäßen abwinkelbaren Rohres können beispielsweise als Spritzgußteile in großer Stückzahl kostengünstig hergestellt werden, wobei die Anordnung von Nasen und Ausnehmungen so vorzunehmen ist, daß eine Montage durch elastisches Aufbiegen von Zungen oder Ausnehmungen möglich ist.

In der Regel weitaus günstiger wird jedoch das Verfahren zur Herstellung eines solchen abwinkelbaren Rohres sein, wie es beispielsweise als flexibler Endoskopschaft, als Hohlwelle, als Kette, als flexibler Trokar, als Stent oder dergleichen einsetzbar ist. Demgemäß wird ein starres Rohr durch ein Trennverfahren in die vorbeschriebenen Rohrabschnitte zerteilt. In diesem Fall entfällt eine Montage der Rohrabschnitte zueinander. Das Rohr kann vor Herstellung der Trennfugen einer Oberflächenbearbeitung zugeführt werden, was die Fertigung weiter vereinfacht. Bei geeignetem Trennverfahren wird es in der Regel nicht erforderlich sein, die einzelnen Rohrabschnitte einer Nachbearbeitung zuzuführen.

Das Herstellen der Trennfugen kann prinzipiell mit jedem nur denkbaren Schneidverfahren durchgeführt werden. Insbesondere bei dünnwandigen Rohren, wie sie in der Endoskoptechnik einigesetzt werden, wird das Trennen mittels Laserstrahl zu bevorzugen sein, da hiermit eine schnelle und wirtschaftliche Fertigung bei nachbearbeitfreier Trennfuge möglich ist. Bei dickwandigeren Rohren größeren Durchmessers kann die Trennfuge auch durch Fräsen und bei noch größeren Rohren auch durch Brennen erfolgen. Ein weiteres Trennverfahren wird das chemische oder elektrochemische Trennen sein. Auch hierbei wird eine Nachbearbeitung der Trennfuge in der Regel entbehrlich sein.

Die Erfindung ist nachfolgend anhand von in der Zeichnung dargestellten Ausführungsbeispielen näher erläutert. Es zeigen:
- Fig. 1: in Seitenansicht zwei zusammenhängende, durch ein Trennverfahren hergestellte Rohrabschnitte einer ersten erfindungsgemäßen Ausführungsforin;
- Fig. 2: einen Schnitt durch die in Figur 1 dargestellten Abschnitte längs der Schnittlinie II-II;
- Fig. 3a: eine zweite erfindungsgemäße Ausführungsform zweier mit einem Trennverfahren hergestellter Abschnitte eines flexiblen Schafts in einer ersten gestreckten Stellung;
- Fig. 3b: die Ausführungsform nach Fig. 3a in einer zweiten gestreckten Stellung,
- Fig. 3c: eine Ausführungsvariante dieser zweiten Ausführungsform in Darstellung nach Fig. 3a,
- Fig. 4: eine dritte, mit einem Trennverfahren hergestellte Ausführung zweier formschlüssig zusammenhängender Abschnitte im Längsschnitt,
- Fig. 5: die beiden Abschnitte gemäß Figur 4 in Seitenansicht,
- Fig. 6: die Abschnitte gemäß Figur 4 im abgewinkeltem Zustand und Schnittdarstellung nach Fig. 4,
- Fig. 7: eine vierte, mit einem Trennverfahren hergestellte Ausführung von drei formschlüssig zusammenhängenden Abschnitten im Längsschnitt (Schnittlinie VII-VII in Fig. 9) in gestreckter Anordnung,
- Fig. 8: die Ausführung nach Fig. 7 in abgewinkelter Stellung und Darstellung nach Fig. 7,
- Fig. 9: die Ausführung nach Fig. 7 in um 90° um die Längsachse gedrehter Schnittdarstellung (Schnittlinie IX-IX in Fig. 7) und
- Fig. 10: die Anordnung nach Fig. 7 in abgewinkelter Stellung und Darstellung nach Fig. 9.

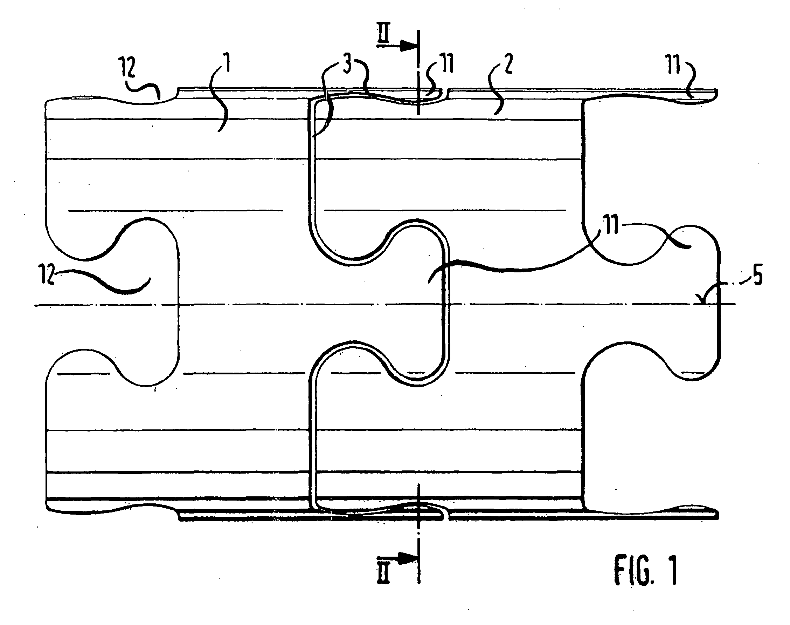

Bei dem Trennverfahren wird in einer in sich geschlossenen Bahn eine Trennfuge in ein starres Rohr geschnitten, was vorzugsweise mittels Laserstrahl erfolgt. Durch das Laserstrahltrennen läßt sich in ein starres Rohr beispielsweise eine mäanderförmige Trennfuge, wie die in Figur 1 gezeigte Fuge 3 so schneiden, daß zusammenhängende Rohrabschnitte 1 und 2 entstehen (in Figur 1 sind nur zwei solcher Rohrabschnitte dargestellt). Dabei ist es von Vorteil, daß der einzelne Rohrabschnitt bereits mit Beendigung des Laserschneidvorgangs, also ohne jede weitere Nachbearbeitung fertig bearbeitet und montiert ist, und jeder einzelne Abschnitt eine homogene Wandstärke aufweist.

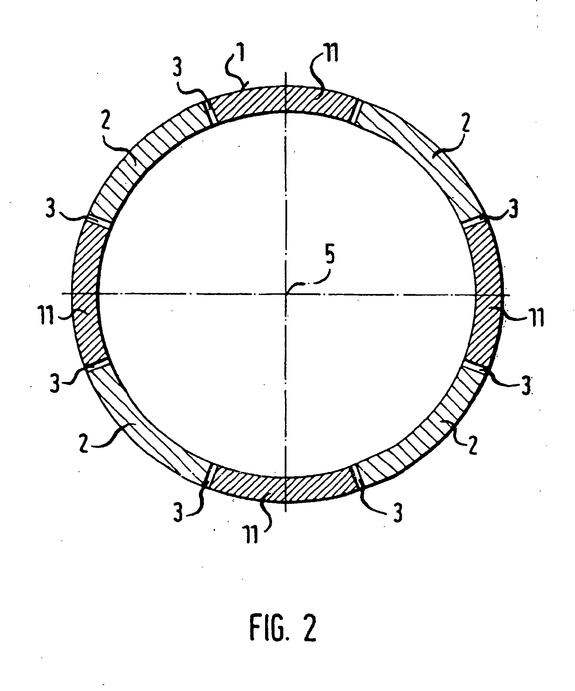

In Verbindung mit dem in Figur 2 gezeigten Querschnitt durch die beiden Rohrabschnitte 1 und 2 längs der in Figur 1 gezeigten Schnittlinie II-II läßt sich deutlich erkennen, daß die auf der Höhe des Schnitts II-II sichtbare Schnittfuge 3 in Umfangsrichtung gesehen an einem Rohrabschnitt vier gegeneinander um 90° versetzte Nasen 11 und vier ebenfalls um 90° gegeneinander versetzte Ausnehmungen 12 bildet, die im wesentlichen dieselbe pilzförmige Kontur wie die Nasen 11 haben. Bei jedem Abschnitt 1,2 liegt jeder Nase 11 eine jeweilige Ausnehmung 12 in axialer Richtung gegenüber. Die Ausnehmungen bilden in Umfangsrichtung gesehen jeweils Hinterschneidungen der Nasen. Dadurch können die einzelnen Abschnitte nicht ohne Beschädigung voneinander getrennt werden. Der Grad der Flexibilität ist von der Breite der Trennfuge 3, von der Länge und vom Durchmesser des einzelnen Elementes abhängig.

Selbstverständlich ist die Anzahl der zu einer Seite vorgesehenen Nasen 11 und Ausnehmungen 12 nicht auf die Zahl 4 beschränkt. Durch vier einander abwechselnde Nasen und Ausnehmungen läßt sich eine sehr gute flexible Auslenkung in allen Ebenen im Raum erreichen.

Auch können die Trennfugen eines mehrere Abschnitte umfassenden Rohrabschnitts so ausgebildet sein, daß einander in axialer Richtung benachbarte Nasen und Ausnehmungen in Umfangsrichtung um einen bestimmten Winkelbetrag versetzt sind.

Mit Hilfe der in den Figuren 1 und 2 dargestellten Unterteilung eines Schaftrohrs können flexible Endoskopschäfte, flexible Trokarhülsen, flexible Zangen, flexible Techno- und Endoskope sowie beispielsweise flexible Schäfte für Kardanwellen in Knochen und Knorpelfräsen oder die Kardanwellen selbst hergestellt werden.

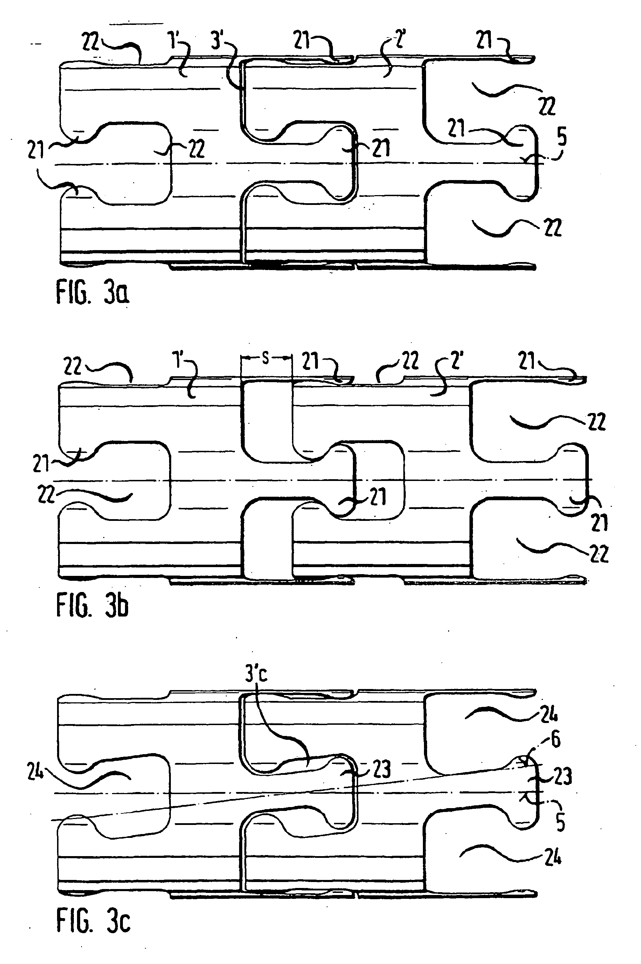

Bei der in Figur 3a dargestellten Ausführung ist die Trennfuge 3' zwischen zwei benachbarten identischen Rohrabschnitten 1',2' so ausgeführt, daß die in Ausnehmungen 22 liegenden Nasen 21 durch die Form ihrer Hinterschneidungen einen definierten Abstand voneinander haben, so daß ein definiertes Spiel S in axialer Richtung gewährleistet ist. Ein so ausgeführter flexibler Endoskopschaft kann dadurch in seiner Länge variiert werden und ist z.B. in Verbindung mit Instrumenten vorteilhaft, die unter Verwendung von wechselweise aufblasbaren und entlüftbaren ringförmigen Manschetten wurmförmige Bewegungen ausführen, um unter Zuhilfenahme dieser Instrumentenbewegung beispielsweise leichter in den Darm oder andere Körperkanäle eingeführt werden zu können. Die Figuren 3a und 3b zeigen die beiden Abschnitte 1' und 2' in den beiden Endstellungen, in denen der flexible Schaft seine kürzeste bzw. längste Längenausdehnung hat. Allerdings ist beim Einsatz solcher in Figur 3a gezeigter Rohrabschnitte 1', 2' gegebenenfalls ein äußerer Schaft oder ein anderes geeignetes Führungsmittel erforderlich, um zu verhindern, daß einander benachbarte Abschnitte außer Eingriff gelangen können, was beispielsweise bei einer Abwinkelung möglich ist. Verhindert werden kann dies beispielsweise auch durch ein kurzes dünnwandiges Ringzylinderteil entweder außerhalb oder innerhalb der ineinander eingreifenden Rohrabschnitte.

Bei der anhand von Fig. 3c dargestellten Ausführung ist zwischen den Hintergreifungen, welche benachbarte Rohrabschnitte in Längsrichtung des Rohres formschlüssig miteinander verbinden, nicht nur ein axiales Spiel S vorgesehen, sondern darüberhinaus sind die Nasen 23 und die korrespondierenden Ausnehmungen 24 in bezug auf die Längsachse 5 des Rohres schräggestellt, derart, daß eine gedachte Verlängerung 6 dieser Nasen 23 bzw. Ausnehmungen 24 schraubenlinienförmig das Rohr umfährt. Bei einer solchen Anordnung dient dann das axiale Spiel S weniger einem Längenausgleich, ähnlich einer Kerbverzahnung oder dergleichen, sondern zur Erzeugung einer Drehbewegung. Werden nämlich die Rohrabschnitte von der in Fig. 3c dargestellten kürzesten Endstellung in die längste Endstellung verfahren, bei der die seitlichen Vorsprünge der Nasen 23 an denen der Ausnehmungen 24 anliegen, dann erfolgt eine Abwinkelung des Rohres um seine Längsachse 5 herum, und zwar zwischen den endseitigen Rohrabschnitten. Diese Abwinkelung um die Längsachse 5 kann zum Erzeugen einer gezielten Drehbewegung genutzt werden, wenn innerhalb oder außerhalb des Rohres entsprechende Zug- und/oder Druckmittel vorgesehen werden. Mit einer solchen Anordnung kann beispielsweise ein Instrument innerhalb eines Schaftes gedreht werden, ohne daß eine Drehbewegung zur Steuerung erforderlich ist. Die Trennfuge 3'c ist also gegenüber der Trennfuge 3' im Bereich ihrer Längserstreckung schräg zur Achse 5 angeordnet.

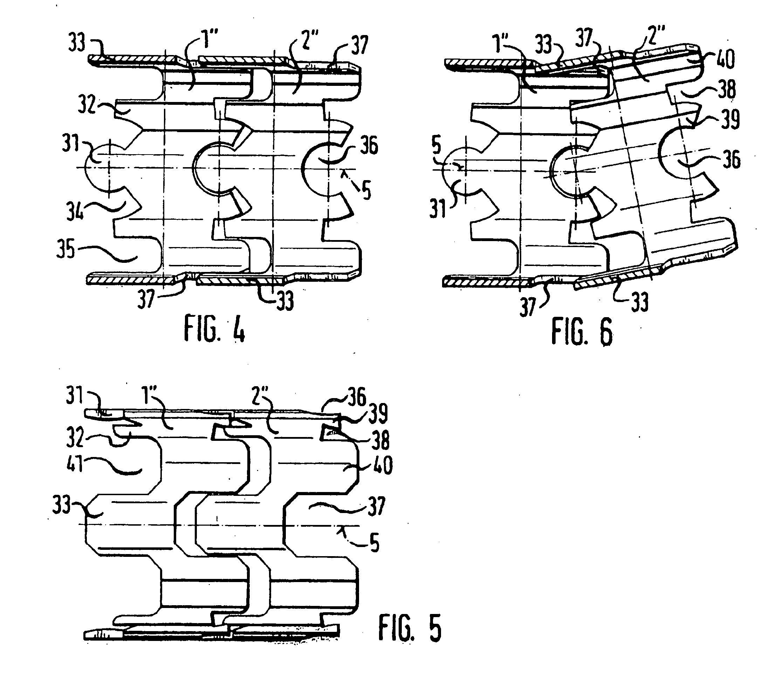

Die Figuren 4 bis 6 zeigen zwei formschlüssig ineinandereingreifende identische Rohrabschnitte 1", 2" einer dritten Ausführungsform, die aus einem starren Rohr geschnitten sind. Beide Rohrabschnitte 1", 2" weisen zwei in einer axialen Richtung ausgebildete erste Nasen 31 mit teilkreisförmiger Kontur auf, die gegeneinanderin Umfangsrichtungum 180° versetzt angeordnet sind. In der gleichen axialen Richtung weisen die Rohrabschnitte zweite Nasen 33 mit rechteckiger Kontur auf, die in Umfangsrichtung ebenfalls um 180° versetzt angeordnet und gegenüber den ersten teilkreisförmigen Nasen 31 jeweils um 90° versetzt sind. Ferner weisen beide Rohrabschnitte 1'', 2" jeweils zwischen den ersten teilkreisförmigen Nasen 31 und den zweiten rechteckförmigen Nasen 33, Ausnehmungen 34, 35 auf, sowie eine zwischen beiden Ausnehmungen angeordnete Nase 32, die bei entsprechender Dimensionierung der Nasen 31, 33 gegebenenfalls auch entfallen kann. Die Ausnehmung 34 hinterschneidet die ersten Nasen 31. In der anderen axialen Richtungweisen beide Rohrabschnitte 1",2" erste Ausnehrnungen 36 mit teilkreisförmiger Kontur auf, die den ersten Nasen 31 axial gegenüberliegen. Ferner weisen beide Rohrabschnitte 1", 2" den rechteckförmigen zweiten Nasen 33 gegenüberliegendezweite rechteckige Ausnehmungen 37 auf sowie zusätzliche die Nasen 32 aufnehmende Ausnehmungen 38 auf.

Wie Figur 6 zeigt, lassen sich die beiden Abschnitte 1" und 2" zueinander abwinkeln, wobei die teilkreisförmigen ersten Nasen 31 des zweiten Abschnittes 2" formschlüssig in den teilkreisförmigen ersten Ausnehmungen 36 des ersten Abschnittes 1" sowie die zweiten Nasen 33 bzw. 40 in den rechteckförmigen Ausnehmungen 37 bzw. 41 verschwenkbar sind ohne daß diese außer Eingriff gelangen können. Somit kann ein flexibler Schaftabschnitt, der mit Rohrabschnitten 1", 2" gemäß den Figuren 4 bis 6 versehen ist, beispieisweise für ein distal steuerbares Endteil bei flexiblen Techno- und Endoskopen Verwendung finden, wenn eine Abwinkelung in nur einer Ebene gewünscht ist.

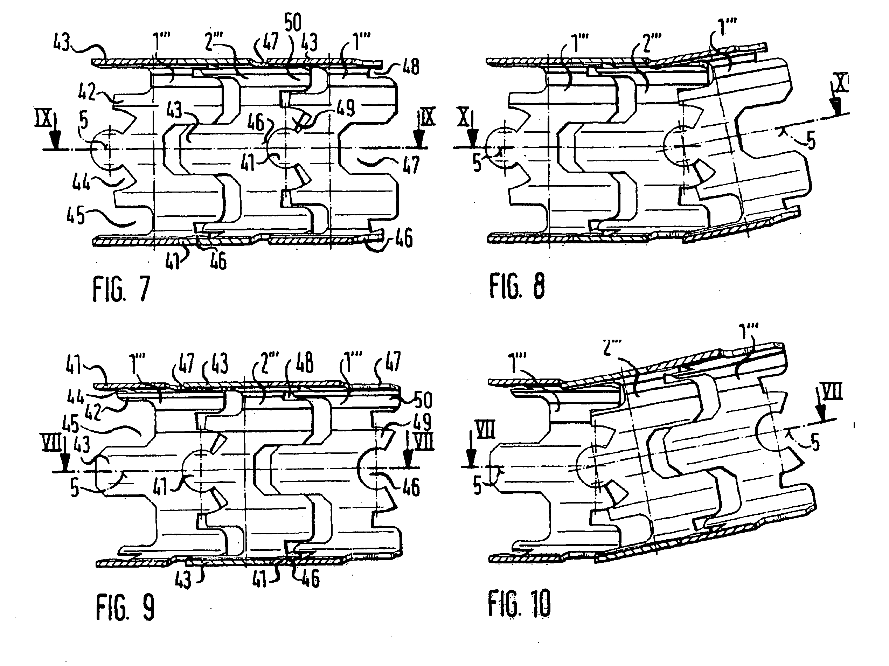

Die anhand der Figuren 7 bis 10 dargestellten Ausführungsvariante unterscheidet sich von der anhand der Figuren 4 bis 6 dargestellten dadurch, daß die Ausnehmungen eines Rohrabschnittes zu den korrespondierenden Nasen desselben Rohrabschnittes nicht achsgleich, sondern um 90° versetzt zueinander angeordnet sind, so daß aufeinanderfolgende Rohrabschnitte stets abwechselnd in anderen Ebenen abwinkelbar sind.

In den Figuren 7 bis 10 sind die benachbarten Rohrabschnitte mit 1''' und 2''' bezeichnet, wobei jeweils drei Rohrabschnitte dargestellt sind. Jeder Rohrabschnitt weist in den Figuren linksseitig zwei im wesentlichen rechteckige Nasen 43 auf, die um 180° versetzt zueinander angeordnet sind. Dazu jeweils um 90° versetzt sind teilkreisförmige Nasen 41. Zwischen den Nasen 41 und 43 sind Nasen 42 angeordnet, die bei entsprechender Dimensionierung entsprechend den Nasen 32 der dritten Ausführungsform gegebenenfalls entfallen können. Zwischen den Nasen 41 und 42 sind Ausnehmungen 44 gebildet, zwischen den Nasen 42 und 43 Ausnehmungen 45.

Zu der in Figuren 7 bis 10 rechten Seite weisen die Rohrabschnitte im wesentlichen rechteckige Nasen 50 sowie dazwischen angeordnete Nasenpaare 49 auf. Zwischen zwei Nasen 49 ist eine teilkreisförmige Ausnehmung 46 gebildet, zwischen zwei benachbarten Nasen 50 eine rechteckige Ausnehmung 47. Die um 180° versetzt zueinander angeordneten teilkreisförmigen Ausnehmungen 46 dienen zur Aufnahme der Nasen 41 des rechts benachbarten Rohrabschnittes. Diese Verbindung zwischen den Nasen 41 und den Ausnehmungen 46 bildet eine Gelenkfunktion um die gemeinsame Mittelpunktachse der Nasen 41. Darüberhinaus wird durch diese Formschlußelemente ein Formschluß in Richtung der Längsachse 5 des Rohres bzw. des Rohrabschnittes erreicht. Die rechteckigen Nasen 43 bilden zusammen mit den rechteckigen Ausnehmungen 47 Führungen, welche die Torsionssteifigkeit des Rohres im gestreckten und abgewinkelten Zustand erhöhen.

Dadurch, daß die zu den Nasen 41 korrespondierenden Ausnehmungen 46 bzw. die zu den Nasen 43 korrespondierenden Ausnehmungen 47 desselben Rohrabschnittes um 90° zueinander versetzt sind, ergibt sich abwechselnd eine Abwinkelbarkeit in einer und nachfolgend in einer dazu um 90° versetzten Ebene, wodurch bei genügender Anzahl von Rohrabschnitten ein in allen Richtungen flexibles Rohr geschaffen wird, das eine hohe Eigenstabilität bei kompaktem Aufbau aufweist. Der sich im Vergleich zu der dritten Ausführungsform (Fig. 4 bis 6) möglicherweise in einer Ebene ergebene größere Biegeradius des Rohres kann zumindest teilweise durch eine Verkürzung der Rohrabschnitte ausgeglichen werden, wie es aufgrund der versetzten Anordnung von Ausnehmungen und Nasen möglich ist.

Allen vorbeschriebenen Ausführungsformen ist gemeinsam, daß die für die Beweglichkeit der einzelnen Rohrabschnitte verantwortliche Trennfuge durch einen Laserstrahlschneideprozeß aus einem starren Schaftrohr geschnitten wird. Selbstverständlich ist die Erfindung nicht auf die anhand der Figuren 1 bis 10 beschriebenen Ausführungsformen beschränkt. Wenn z.B. die Trennfuge so gestaltet ist, daß sie aus dem starren Schaft keine geraden Rohrabschnitte sondern schräge Rohrabschnitte ausschneidet, kann dadurch eine Ausrichtung der flexiblen Beweglichkeit des Schaftabschnitts oder eine bestimmte Krümmung desselben vorgegeben werden.

Ferner können z.B. Rohrabschnitte gemäß Figur 1 mit solchen gemäß Figur 3a oder auch den Figuren 4 bis 10 in einem beweglichen Schaftteil kombiniert sein.

## Patentansprüche

1. Abwinkelbares Rohr, insbesondere als Schaft für ein flexibles Endoskop, bestehend aus Rohrabschnitten (1, 2), von denen benachbarte Rohrabschnitte (1, 2) durch eine umlaufende Trennfuge (3) materiell voneinander getrennt jedoch formschlüssig mindestens in Richtung der Längsachse (5) des Rohres miteinander verbunden sind und ein Rohrabschnitt (1) vier oder mehr zum benachbarten Rohrabschnitt (2) weisende Nasen (11) aufweist, wobei mindestens eine Nase (11) in eine entsprechende Ausnehmung (12) des benachbarten Rohrabschnitts (2) formschlüssig eingreift.

2. Rohr nach Anspruch 1, **dadurch gekennzeichnet, dass** eine Ausnehmung (12) durch den zwischen zwei benachbarten Nasen (11) desselben Rohrabschnitts gebildeten Zwischenraum gebildet ist.

3. Rohr nach einem der vorhergehenden Ansprüche, **dadurch gekennzeichnet, dass** eine Nase (11) eine gerundete pilzförmige, vorzugsweise etwa teilkreisförmige Außenkontur aufweist.

4. Rohr nach einem der vorhergehenden Ansprüche, **dadurch gekennzeichnet, dass** eine Nase (33, 40) eine rechteckige Außenkontur aufweist.

5. Rohr nach einem der vorhergehenden Ansprüche, **dadurch gekennzeichnet, dass** ein Rohrabschnitt (1") zu einer Seite zwei um 180°versetzt angeordnete Nasen (31) teilkreisförmiger Kontur und zwei jeweils um 90° dazu versetzt angeordnete Nasen (33) rechteckiger Kontur aufweist sowie entsprechend geformte Ausnehmungen (35, 36) zur anderen Seite.

6. Rohr nach einem der vorhergehenden Ansprüche, **dadurch gekennzeichnet, dass** die Nasen (49, 50) zu einer Seite eines Rohrabschnitts (1''') bezogen auf die entsprechenden Ausnehmungen (44, 45) zur anderen Seite dieses Rohrabschnitts (1''') um einen Winkel versetzt angeordnet sind.

7. Rohr nach einem der vorhergehenden Ansprüche, **dadurch gekennzeichnet, dass** die Nasen (21) und die zugehörigen Ausnehmungen (22) im Bereich ihrer Hintergreifungen einen Abstand (s) aufweisen.

8. Rohr nach einem der vorhergehenden Ansprüche, **dadurch gekennzeichnet, dass** die Nasen (23) und die zugehörigen Ausnehmungen (24) so ausgebildet und angeordnet sind, daß sich deren gedachte Längsachse (6) schraubenlinienförmig um die Längsachse (5) des Rohres erstreckt.

## Claims

1. A tube able to be angled, in particular as a shank for a flexible endoscope, consisting of tube sections (1, 2) of which adjacent tube sections (1,2) are separated materially from one another by a peripheral separating gap (3) but are connected to one another with a positive fit at least in the direction of the longitudinal axis (5) of the tube, and a tube section (1) comprises four or more lugs (11) directed to the adjacent tube section (2), wherein at least one lug (11) engages into the corresponding recess (12) of the adjacent tube section (2) with a positive fit.

2. A tube according to claim 1, **characterised in that** a recess (12) is formed by the intermediate space formed between two adjacent lugs (11) of the same tube section.

3. A tube according to one of the preceding claims, **characterised in that** a lug (11) has a rounded, mushroom-like, preferably roughly part-circular outer contour.

4. A tube according to one of the preceding claims, **characterised in that** a lug (33, 40) has a rectangular outer contour.

5. A tube according to one of the preceding claims, **characterised in that** a tube section (1") to one side comprises two lugs (31) of a part-circular contour which are offset by 180° and two lugs (33) of a rectangular contour which in each case are offset to these by 90°, as well as correspondingly shaped recesses (35, 36) to the other side.

6. A tube according to one of the preceding claims, **characterised in that** the lugs (49, 50) to one side of a tube section (1"') are arranged offset by an angle with respect to the corresponding recesses (44, 45) to the other side of this tube section (1"').

7. A tube according to one of the preceding claims, **characterised in that** the lugs (21) and the associated recesses (22) have a distance (s) in the region of their undercuts.

8. A tube according to one of the preceding claims, **characterised in that** the lugs (23) and the associated recesses (24) are designed and arranged such that their imagined longitudinal axis (6) extends in a helical manner around the longitudinal axis (5) of the tube.

## Revendications

1. Tube pouvant être coudé, notamment en tant que corps tubulaire pour un endoscope flexible, composé de tronçons de tube (1, 2) dont des tronçons de tube voisins (1,2) sont séparés l'une de l'autre, sur le plan de la continuité de matière, par un joint de séparation (3) rotatif, mais sont toutefois reliés l'un à l'autre par complémentarité de forme au moins dans la direction de l'axe longitudinal (5) du tube, un tronçon de tube (1) présentant quatre talons (11) ou davantage dirigés vers le tronçon de tube voisin (2), et au moins un talon (11) s'engageant par complémentarité de forme dans un évidement (12) correspondant du tronçon de tube voisin (2).

2. Tube selon la revendication 1, **caractérisé en ce qu'**un évidement (12) est formé par l'espace intermédiaire entre deux talons (11) voisins du même tronçon de tube.

3. Tube selon l'une des revendications précédentes, **caractérisé en ce qu'**un talon (11) présente un contour extérieur arrondi, en forme de champignon, de préférence sensiblement de forme partiellement circulaire.

4. Tube selon l'une des revendications précédentes, **caractérisé en ce qu'**un talon (33, 40) présente un contour extérieur de forme rectangulaire.

5. Tube selon l'une des revendications précédentes, **caractérisé en ce qu'**un tronçon de tube (1") présente, vers un côté, deux talons (31) d'un contour de forme partiellement circulaire, disposés de manière décalée de 180°, et, disposés de manière décalée respectivement de 90° par rapport à ceux-ci, deux talons (33) de contour rectangulaire, ainsi que des évidements (35, 36) de forme correspondante, vers l'autre côté.

6. Tube selon l'une des revendications précédentes, **caractérisé en ce que** les talons (49, 50) vers un côté d'un tronçon de tube (1"') sont disposés de manière décalée d'un angle par rapport aux évidements correspondants (44, 45) vers l'autre côté de ce tronçon de tube (1"').

7. Tube selon l'une des revendications précédentes, **caractérisé en ce que** les talons (21) et les évidements (22) associés présentent une distance d'espacement (s) dans la zone de leurs contre-dépouilles.

8. Tube selon l'une des revendications précédentes, **caractérisé en ce que** les talons (23) et les évidements (24) associés sont conçus et disposés de façon telle, que leur axe longitudinal fictif (6) s'étende selon une ligne en forme d'hélice autour de l'axe longitudinal (5) du tube.
